# EUROPÄISCHE PATENTANMELDUNG

(11) **EP 1 969 995 A1**
(43) Veröffentlichungstag der Anmeldung: **17.09.2008**
(21) Anmeldenummer: 07405084.0
(22) Anmeldetag: 14.03.2007
(51) Int. Cl.: A61B 3/12

(54) **Augenuntersuchungsvorrichtung**

(71) Anmelder: HAAG-STREIT AG, CH-3098 Köniz (CH)
(72) Erfinder: Wälti, Rudolf, Dr., 3097 Liebefeld (CH); Walker, Jürg, 3054 Schüpfen (CH)
(74) Vertreter: Rüfenacht, Philipp Michael

(57) **Zusammenfassung**

Eine Untersuchungsvorrichtung zur Untersuchung eines Auges umfasst ein Spaltlampenmikroskop sowie ein SDO (scanning digital ophthalmoscope) zur Erzeugung einer Abbildung eines ersten Augenabschnittes. Weiter umfasst die Vorrichtung ein SDOCT (spectral domain optical coherence tomograph), mit welchem eine Schnittansicht eines zweiten Augenabschnittes parallel zu einer optischen Achse der Untersuchungsvorrichtung erzeugbar ist.

## Beschreibung

### Technisches Gebiet

Die Erfindung betrifft eine Untersuchungsvorrichtung zur Untersuchung eines Auges mit einer ersten Vorrichtung zur Erzeugung einer Abbildung des Auges, wobei die erste Vorrichtung ein Mikroskop sowie eine Beleuchtungseinheit zur Beleuchtung des Auges umfasst. Weiter betrifft die Erfindung ein Verfahren zur Untersuchung eines Auges mit einer Untersuchungsvorrichtung, wobei mit einer ersten Vorrichtung eine Abbildung des Auges erzeugt wird, indem das Auge mit einer Beleuchtungseinheit beleuchtet und mit einem Mikroskop die Abbildung erzeugt wird.

### Stand der Technik

Für die Beobachtung und Dokumentierung des Fundus werden verschiedene physikalische Verfahren angewandt. Bei der Fundusfotografie oder bei Untersuchungen mit einem SDO wird nur reflektiertes Licht von der Oberfläche des Fundus erfasst. Tiefer liegende Strukturen können durch die Verwendung von unterschiedlichen Wellenlängen der Beleuchtung oder konfokalen Optiken dargestellt werden. Der Arzt kann jedoch schon aufgrund der Bildinformation von der Oberfläche eine Vielzahl von Pathologien erkennen. Zusätzliche Informationen können der Topographie des Fundus entnommen werden. Dies wird beispielsweise bei der Verwendung eines Binokulartubus, welcher einen stereoskopischen Eindruck vermittelt, ermöglicht.

Oft besteht jedoch das Bedürfnis, auch tiefer liegende Strukturen des Fundus darzustellen. Dies ist mit Untersuchungsgeräten möglich, welche beispielsweise die OCT-Technologie anwenden. Die neuere SDOCT-Technologie kann für diesen Zweck ebenfalls eingesetzt werden.

Sollen bei einem Patienten sowohl der Fundus als auch entsprechend tieferliegende Strukturen des Fundus untersucht werden, müssen hierfür verschiedene Untersuchungsgeräte benutzt werden. Entsprechend muss der Patient auch an verschiedenen Untersuchungsgeräten platziert werden. Dies ist sowohl kosten- als auch zeitintensiv und zudem für den Patienten unangenehm.

### Darstellung der Erfindung

Aufgabe der Erfindung ist es, eine dem eingangs genannten technischen Gebiet zugehörende Vorrichtung zu schaffen, welche eine vereinfachte Untersuchung verschiedener Augenabschnitte, insbesondere des Fundus und der tieferliegenden Fundusstrukturen, ermöglicht.

Die Lösung der Aufgabe ist durch die Merkmale des Anspruchs 1 definiert. Eine Untersuchungsvorrichtung zur Untersuchung umfasst eine erste Vorrichtung zur Erzeugung einer Abbildung des Auges, wobei die erste Vorrichtung ein Mikroskop sowie eine Beleuchtungseinheit zur Beleuchtung des Auges umfasst. Gemäss der Erfindung umfasst die Untersuchungsvorrichtung eine zweite Vorrichtung zur Erzeugung einer Abbildung eines ersten Augenabschnittes sowie eine dritte Vorrichtung zur Erzeugung einer tiefenaufgelösten Abbildung eines zweiten Augenabschnittes. Mit der zweiten Vorrichtung ist die Abbildung des ersten Augenabschnitts mittels einer sequentiellen Abtastung des ersten Augenabschnitts mit einem Lichtstrahl erzeugbar und mit der dritten Vorrichtung ist eine Schnittansicht des zweiten Augenabschnittes parallel zu einer optischen Achse der Untersuchungsvorrichtung erzeugbar. Hierbei kann der zweite Augenabschnitt vom ersten Augenabschnitt verschieden sein. Vorzugsweise ist der zweite Augenabschnitt jedoch ein Teilbereich des ersten Augenabschnitts.

Bei der ersten Vorrichtung handelt es sich insbesondere um ein sogenanntes Spaltlampenmikroskop mit einer entsprechenden, seitlichen Spaltbeleuchtung, mit welcher ein schmales, spaltförmiges Lichtbündel erzeugbar ist.

Die zweite Vorrichtung ist insbesondere ein sogenanntes SDO (scanning digital ophthalmoscope) und die dritte Vorrichtung ist insbesondere ein OCT (optical coherence tomograph), d. h. eine Vorrichtung zur Durchführung einer optischen Kohärenztomographie. Vorzugsweise handelt es sich hierbei um die moderne spektrale optische Kohärenztomographie (SDOCT - spectral domain optical coherence tomography). Das Kombinieren eines SDO's mit einer Spaltlampe bringt allein schon den Vorteil, dass die scannende, reflexarme Beleuchtung, zusammen mit der Beobachtung durch den Binokulartubus, einen guten stereoskopischen Eindruck des Fundus ermöglicht.

Die gemeinsame Verwendung des SDOs und eines SDOCTs erlaubt eine erweiterte Untersuchungsmöglichkeit, welche auf dem gleichen Untersuchungsgerät durchgeführt werden kann. Das SDO liefert dem Untersucher erste wichtige Informationen zu möglichen Pathologien auf der Netzhaut. Das SDOCT erlaubt nun das Scannen von interessierenden Stellen auf dem Fundus, ohne dass der Patient dafür an ein anderes Untersuchungsgerät umplatziert werden muss. Der zu scannende Bereich wird mit einer auf dem Fundus projizierten Linie sichtbar gemacht. Mit der Umlenkeinheit **40** wird die Position bestimmt. Als Lichtquelle für die Fixationshilfe des Patienten dient vorzugsweise eine sichtbare Laserquelle **2,** welche über den 2x2 Koppler **162** in die Faser **14b** und damit auf dem gleichen Strahlengang wie der Messstrahl des SDOCT auf den zu untersuchenden Bereich gelangt. Fixationslaser 2 und Messlaser 1 sind somit immer kongruent. Der Zielbereich des Messlasers könnte in einer alternativen Lösung auch synthetisch, abhängig von der Position des Translators durch die Software berechnet, dem SDO-Bild überlagert werden.

B-Scans sind auch für den vorderen Augenabschnitt, insbesondere im Bereich der hinteren Augenkammer und der Ziliarkörper, interessant (Kammerwinkel). Für Untersuchungen im vorderen Bereich des Auges darf die Ophthalmoskopierlinse **13** nicht zwischengeschaltet werden.

### Kurze Beschreibung der Zeichnungen

Die zur Erläuterung des Ausführungsbeispiels verwendeten Zeichnungen zeigen:
- Fig. 1: eine schematisch dargestellte Untersuchungsvorrichtung gemäss der Erfindung;
- Fig. 2a: eine schematische Abbildung eines Augenfundus mit einer Markierung;
- Fig. 2b: eine schematische Abbildung eines zweidimensionalen Tiefenscans der in Figur 2a markierten Region der Fundusabbildung aus Fig. 2a;
- Fig. 3: eine Ausführungsform der erfindungsgemässen Untersuchungsvorrichtung mit 8 LEDs zur Messung des Abstands zwischen der Sehachse und dem Pupillenschwerpunkt;
- Fig. 4a: einen softwaremässig erzeugten Kreis mit grossem Durchmesser als Positionierhilfe bei einem schwachen Messsignal und
- Fig.4b: einen softwaremässig erzeugten Kreis mit kleinem Durchmesser als Positionierhilfe bei einem starken Messsignal.

Grundsätzlich sind in den Figuren gleiche Teile mit gleichen Bezugszeichen versehen.

### Wege zur Ausführung der Erfindung

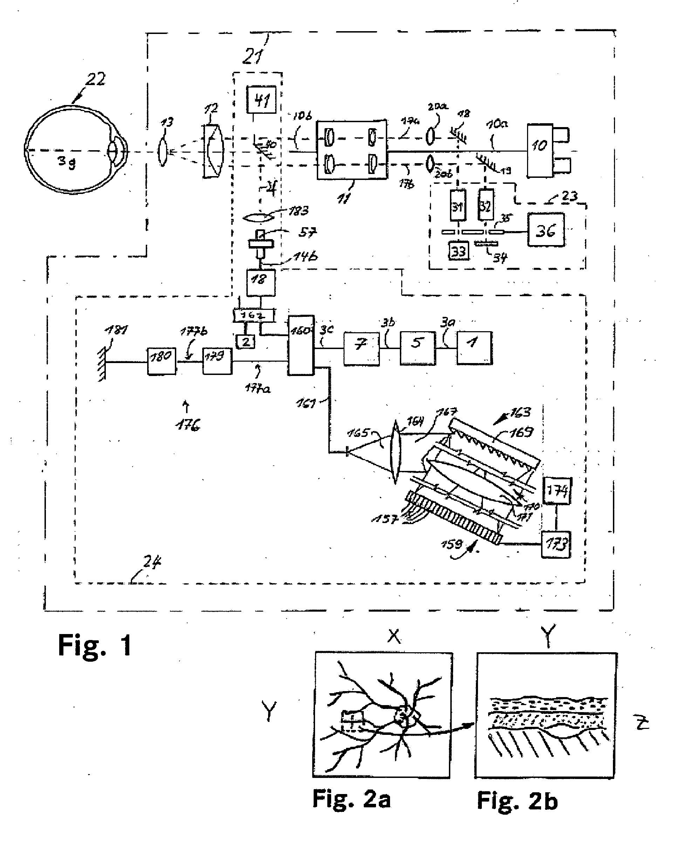

**Fig.1** zeigt den Gesamtaufbau des Untersuchungsgerätes, mit einem typischen Strahlengang eines Spaltlampenmikroskops zur Untersuchung des vorderen und hinteren Augenabschnittes eines Auges. Über das Binokular **10** führen zwei parallel laufende Strahlengänge **10a** zu einem 2-Achsen-Vergrösserungswechsler **11.** Beide der parallel laufenden Strahlen weisen einen auf unendlich eingestellten Fokus auf. Von dort gelangt das weiterhin parallel verlaufende Strahlenpaar 10b zur Frontlinse **12** des Mikroskops. Die Frontlinse **12** fokussiert die Strahlen auf der Augenhornhaut des Objektes **22.** Durch ein optionales Zwischenschalten einer Ophthalmoskopierlinse **13** und entsprechend optimierten Abstand zum Auge liegt der Fokuspunkt auf dem Augenhintergrund. In den oben beschriebenen Strahlengang wird über eine bewegliche optische Umlenkeinheit **40** ein SDOCT **24** eingekoppelt. Der Strahl **3f** des SDOCT verläuft zwischen den optischen Beobachtungsstrahlengängen **10b** zum Objekt **22.** Alternativ kann der Strahl des SDOCT auch seitlich versetzt über einen der Beobachtungsstrahlengänge **10b** geführt werden. Der 3-fach Vergrösserungswechsler **11** verfügt über ein um 90 Grad gedrehtes zweites Strahlenpaar. Über dieses zweite Strahlenpaar werden ein Beleuchtungs- und ein Beobachtungsstrahl des SDO **23** über zwei Spiegel **18** und **19** eingekoppelt. Mit einer zwischen Spiegel **18** bzw. **19** und Vergrösserungswechsler (Galilei-Optik) **11** optional eingefügten Linsenkombination **20a** bzw. **20b,** können die Strahlenbündel der beiden optischen Systeme **11** und **31** bzw. **32** optimal aneinander angepasst werden. Die beiden Strahlen **17a** und **17b** werden durch den Galileiwechsler **11** geführt, wo je nach Drehposition desjenigen unterschiedliche Vergrösserungen entstehen. In direkter Fortsetzung des Galileiwechslers **11** führen die parallel laufenden Strahlengänge über die Frontlinse **12** des Mikroskops und einer alternativ dazwischen geschalteten Ophthalmoskopierlinse **13** zum Objekt **22.**

Das SDO **23** verfügt über zwei Strahlengänge **17a** und **17b.** Das Licht der Lichtquelle **33** gelangt über eine Optik **31** als Lichtbündel **17a** zum Vergrösserungswechsler **11** und auf bereits beschriebenem Weg zum Objekt **22.** Der vom Objekt **22** reflektierte Lichtanteil gelangt auf dem zweiten Strahlengang via den Vergrösserungswechsler **11,** der optional eingesetzten Linsenkombination 20b, den Spiegel **19,** die Linsenkombination **32** zum Bildsensor **34.** Mit der Schwingblende **35** werden störende Streulichter weitgehend eliminiert. Die Bildinformation des Sensors **34** wird einer Auswerteeinheit zugeführt.

Das Spektrometer **163** hat eine Linse **164,** welche die aus dem Strahlungsleiter **161** austretende Strahlung **165** kollimiert. Die kollimierte Strahlung **167** wird auf ein Gitter **169** geführt. Das Gitter **169** reflektiert jede Wellenlänge, die in der auf ein Gitter **169** auftreffenden Strahlung **167** enthalten ist, in eine andere Richtung. Diese nach den Wellenlängenanteilen zerlegte Strahlung **170** wird mittels einer Fokussierlinse **171** auf die die Kamerapixel **157** aufweisende Kamerazeile **159** fokussiert. Jedes Pixel **157** empfängt somit immer nur einen ganz bestimmten Wellenlängenbereich. Mit der Ermittlung der Intensitäten der von den Kamerapixeln detektierten Wellenlängenbereiche und mit Hilfe einer mathematischen Umformung (Fouriertransformation) in einer Auswerteeinheit **173** können die Positionen der verschiedenen Reflexionen im Gegenstand **22** entlang der Ausbreitungsrichtung des Messstrahls **3g** der Strahlungsquelle **1** ermittelt werden.

Der Referenzarm **176** ist über eine Monomodefaser **177a** mit dem Koppler **160** verbunden. Das andere Ende der Monomodefaser **177a** ist mit einem optionalen, z. B. piezoelektrisch arbeitenden Phasenmodulator **179** und dieser über eine Monomodefaser **177b** mit einem optionalen Polarisationskontroller **180** verbunden. Ausgehend von dem Polarisationskontroller **180** gelangt die Strahlung auf einen Spiegel **181,** von dem die Strahlung wieder über die vorgängig angeführten Komponenten zum Koppler **160** zurück reflektiert wird.

Im Faserkoppler **160** wird die vom Gegenstand **22** rückreflektierte Strahlung mit der vom Referenzarm **176** reflektierten Strahlung überlagert. Vom Faserkoppler **160** gehen dann 50 % der Strahlung in den Strahlungsleiter **161.**

Der SDOCT **24** enthält die Strahlungsquelle **1,** den Strahlungsabschwächer **5** (optional), den Polarisationskontroller **7,** den 2x2 Monomodefaserkoppler **160,** das Spektrometer **163,** den Fixationslaser **2** (optional, der Fixationslaser/Pilotlaser **2** ist optional, weil die Strahlungsquelle **1** auch im nahen Infrarotbereich unter Umständen schon genügend sichtbar ist für den Patienten), den Referenzarm **176,** den Polarisationskontroller **18,** die Ferrule **57,** die Linse **183,** die Umlenkeinheit **40,** die Ansteuereinheit **41** für die bewegliche Umlenkeinheit **40,** die Linsenkombination **12** und **13** und den Gegenstand **22.**

Das nach dem Michelson-Prinzip aufgebaute Interferometer kann sowohl faseroptisch als auch als geometrische Optik, d. h. ohne Fasern aber mit einem Strahlenteilerwürfel anstelle eines 2x2 Faserkopplers und einem Freistrahl statt Fasern, aufgebaut werden. Bei der Verwendung von Fasern und der Forderung eines B-Scans ist die Umlenkeinheit **40** beweglich als Translator vorgesehen. Wird ein Translator **40** zusammen mit einem Aufbau ohne Fasern verwendet, kann gar ein 3-D Scan erzielt werden.

In **Fig. 2a** ist ein Fundusbild des SDOs ersichtlich. Eine navigierbare Marke ist sichtbar. Diese wird entweder synthetisch, von der Software, dem SDO-Bild überlagert oder mit einer zusätzlichen LASER-Quelle, entlang des SDOCT-Strahlengangs auf dem Fundus abgebildet.

In **Fig. 2b** ist ein Tiefen-Scan eines SDOCT dargestellt. Die Eindringtiefe ist etwa 3mm. Die Länge des SDOCT B-Scans wird unter anderem durch die Auslenkung der beweglichen Umlenkeinheit **40** bestimmt.

Nochmals zurück zu Figur 1. Zur Messung des Abstands zwischen der Sehachse und dem Pupillenschwerpunkt, während der Patient fixiert, werden mindestens 3 Lichtquellen (vorzugsweise LEDs) in einer Ebene ringförmig um die Frontlinse **12** der Spaltlampe angeordnet. Der Ring dieser Lichtquellen ist konzentrisch zum Messstrahl **3g** des SDOCTs angeordnet. Die Lichtquellen können optional hinter einer Streuscheibe platziert werden. Die Lichtstrahlen der LEDs werden am Tränenfilm des untersuchten Auges **22** reflektiert. Die Reflexion am Tränenfilm führt zur Entstehung von virtuellen Bildern der LEDs **200** innerhalb des Auges. Die Orte der virtuellen Bilder der LEDs befinden sich typischerweise einige Millimeter hinter dem Tränenfilm, je nach Oberflächenkrümmung des Tränenfilms. Das SDO bildet die virtuellen Bilder der LEDs **200** auf den Bildsensor **34** ab.

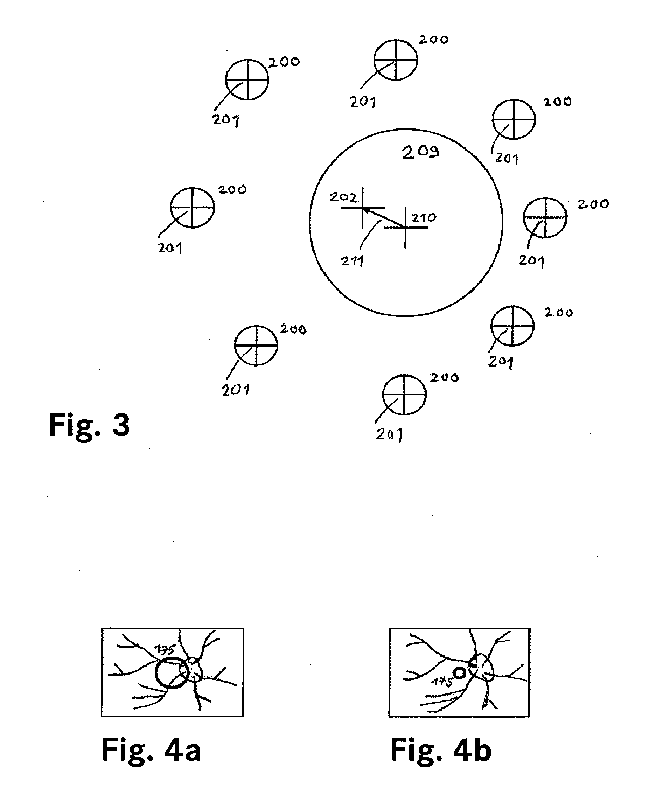

Fig. 3 zeigt eine Ausführung mit 8 LEDs, die auf dem Bildsensor **34** abgebildet sind. Aus den Koordinaten der auf den Bildsensor **34** abgebildeten Schwerpunkte >**201** der LED-Bilder kann ein Zentrum **202** berechnet werden. Mit Hilfe der Lichtquelle **2** wird der Patient angewiesen in eine bestimmte Richtung (Fixationsrichtung) zu blicken. Ob der Patient gut fixiert, kann z. B. überprüft werden, indem das Vorhandensein und die Amplitude der Signale des SDOCT in der Auswerteeinheit **173** ausgewertet werden. Mögliche Strukturen, die das SDOCT misst, sind die Hornhautvorderfläche, Hornhautrückfläche, Linsenvorderfläche, Linsenrückfläche und Retina. Anstelle eines SDOCT kann auch ein Time-Domain OCT (TDOCT) verwendet werden. Sind die reflektierten Signale der gemessenen Strukturen des SDOCT hoch, so kann davon ausgegangen werden, dass der Patient gut fixiert. Wenn der Patient gut fixiert, so geht die Sehachse des Patienten durch das berechnete Zentrum **202** der ringförmig angeordneten LEDs. Zusätzlich zum Zentrum der LEDs wertet der Bildsensor **34** auch den Pupillenschwerpunkt **210** der Pupille **209** aus. Der Vektor der Verbindungslinie **211** zwischen dem Pupillenschwerpunkt **210** und dem berechneten Zentrum der LEDs **202** wird berechnet. Anwendung: Die Länge und die Richtung der Verbindungslinie **211** müssen während der

Planung eines photorefraktiven chirurgischen Eingriffs (z. B. LASIK, LASEK, PRK) berücksichtigt werden, weil das korneale Laserablationsmuster der heute üblichen Lasersysteme auf den Pupillenschwerpunkt **210** zentriert wird. Falls aber die Sehachse nicht durch den Pupillenschwerpunkt **210** geht, was oft der Fall ist, wird dadurch immer ein gewisser Fehler des postoperativen Ergebnisses in Kauf genommen. Grosse Abstände zwischen dem Zentrum der LEDs **202** und dem Pupillenschwerpunkt **210** müssen bei der Ablation berücksichtigt werden, um eine zufriedenstellende postoperative Refraktion des Patienten zu erzielen.

Mit Hilfe der acht LEDs **200** (Bedingung: Es müssen mindestens 5 LEDs sein) kann die Krümmung der Hornhautvorderfläche gemessen werden, indem die auf den Bildsensor **34** abgebildeten Abstände der LED-Schwerpunkte **201** zueinander ausgewertet werden. Dabei gilt, dass die Krümmungsradien mit zunehmendem Abstand zweier LED-Schwerpunkte **201** zunehmen. Die genaue Beziehung zwischen den Krümmungsradien und den LED-Schwerpunkten **201** ist gegeben durch die Position der ringförmig an der Spaltlampe angeordneten LEDs **200,** der Optik und der Geometrie zwischen der Patientenhornhaut und dem Bildsensor **34.** Diese Beziehung kann rechnerisch und/oder mit Hilfe einer Katibrationsmethode ermittelt werden. Insbesondere kann damit der Krümmungsradius des flachen und der Krümmungsradius des steilen Meridians der Hornhautvorderfläche bestimmt werden. Die Differenz zwischen den Krümmungsradien des steilen und flachen Meridians ergibt den Astigmatismus. Die Auswertung der LED-Schwerpunkte auf dem Bildsensor **34** ergibt auch den Winkel Alpha zwischen der horizontalen Achse des Bildsensors **34** und der Achse des flachen oder des steilen Meridians.

Zusätzlich zu den LED-Schwerpunkten **201** wird das auf dem Bildsensor **34** abgebildete Muster der Iris ausgewertet. Die Auswertung des Irismusters soll als Ergebnis die Winkellage Beta einer charakteristischen Struktur im Muster der Iris bezüglich der horizontalen Achse des Bildsensors **34** liefern. Damit kann nun durch einfache Subtraktion (Alpha - Beta) der Winkel Gamma zwischen dem flachen Meridian (oder dem steilen Meridian) und der Winkellage der charakteristischen Irisstruktur berechnet werden. Es wird angenommen, dass der Winkel Gamma für ein und denselben Patienten erstens zeitlich konstant ist und zweitens unabhängig von der Position des Patienten (sitzend oder liegend) ist.

Dieser Winkel Gamma muss bekannt sein z. B. bei der photorefraktiven Ablation z. B. durch einen Excimerlaser. Begründung: Alpha kann während der Excimerlaserbehandlung nicht gemessen werden, weil der Excimerlaser keine dazu notwendige Vorrichtung z. B. in Form von LEDs **200** hat. Beta kann z.B. von der im Excimerlaser integrierten Eyetracker-Kamera online während der Excimerlaserbehandlung gemessen werden. Wird jetzt Beta von der Eyetracker-Kamera gemessen und ist Gamma bekannt, so kann nun auch die Lage des flachen oder steilen Meridians Alpha während der Behandlung ermittelt werden. Weil Alpha in der liegenden Position des Patienten bei der Excimerlaserbehandlung nicht immer identisch ist mit der Winkellage Alpha während der vorgängigen Messung in sitzender Position, ist es bei der ablativen Korrektur eines Astigmatismus durch einen Laser nötig, Gamma zu kennen, ansonsten der Astigmatismus vom Laser falsch korrigiert wird.

Die Anzeigeeinheit **174** (siehe Fig. 1) dient zur Darstellung der Patientendaten, der zur Positionierung benötigten Darstellung des zu messenden Augenbereiches, von softwaremässig erzeugten Marken als Positionierhilfe und der Messergebnisse.

Figuren 4a und 4b zeigen jeweils eine softwaremässig erzeugte Marke als Positionierhilfe. Diese kann z. B. ein vorzugsweise im Zentrum des dargestellten Augenbereiches eingeblendeter Kreis **175** sein, dessen Durchmesser mit zunehmender Signalstärke der von der Auswerteeinheit **173** detektierten Reflexionen des SDOCTs abnimmt. Die Farbe des Kreises kann wechseln sobald die Signale so hoch sind, dass sie als zuverlässig gelten. Anstelle des SDOCTs können die Signale auch von einem Time-Domain OCT stammen. Der Durchmesser des Kreises **175** kann auch anhand der Signalstärke oder Bildschärfe des von dem Bildsensor **34** detektierten Bildes des Fundus oder des vorderen Augenabschnittes geregelt werden. In einer weiteren Ausführungsvariante kann der Durchmesser des Kreises **175** anhand der Signalstärke oder Bildschärfe der von dem Bildsensor **34** detektierten LED-Bilder geregelt werden. Auch eine Regelungskombination der Signalstärken oder Bildschärfen des SDOCT, des SDO-Beobachtungsfeldes und der vom SDO detektierten LED-Bilder kann gewählt werden.

Fig. 4a zeigt einen grossen Kreis **175,** was heisst, dass das Messsignal gering ist. Dadurch weiss der Benutzer, dass er das Messgerät noch besser zum Patientenauge positionieren muss.

Fig. 4b zeigt einen kleinen Kreis **175.** Hier hat der Benutzer das Messgerät besser zum Patientenauge positioniert. Das Messsignal ist stark und damit zuverlässig. Der Benutzer weiss dadurch, dass er die Messung auslösen kann, weil die Signale zuverlässig sind.

## Patentansprüche

1. Untersuchungsvorrichtung zur Untersuchung eines Auges mit einer ersten Vorrichtung zur Erzeugung einer Abbildung des Auges, wobei die erste Vorrichtung ein Mikroskop sowie eine Beleuchtungseinheit zur Beleuchtung des Auges umfasst, **dadurch gekennzeichnet, dass** die Untersuchungsvorrichtung eine zweite Vorrichtung zur Erzeugung einer Abbildung eines ersten Augenabschnittes umfasst und mit der zweiten Vorrichtung die Abbildung des ersten Augenabschnitts mittels einer sequentiellen Abtastung des ersten Augenabschnitts mit einem Lichtstrahl erzeugbar ist, und die Untersuchungsvorrichtung eine dritte Vorrichtung zur Erzeugung einer tiefenaufgelösten Abbildung eines zweiten Augenabschnittes umfasst, mit welcher eine Schnittansicht des zweiten Augenabschnittes parallel zu einer optischen Achse der Untersuchungsvorrichtung erzeugbar ist.

2. Untersuchungsvorrichtung nach Anspruch 1, wobei zur Untersuchung eines hinteren Augenabschnittes mit der ersten Vorrichtung eine Linse nanordnung, insbesondere eine Ophthalmoskopierlinse, zwischen einem Frontobjektiv der Untersuchungsvorrichtung und dem Auge positionierbar ist.

3. Untersuchungsvorrichtung nach Anspruch 1 oder 2, wobei die erste Vorrichtung als Spaltlampenmikroskop, die Beleuchtungseinheit zur Erzeugung eines schmalen, spaltförmigen Lichtbündels, die zweite Vorrichtung als Scanning Digital Ophthalmoskop und die dritte Vorrichtung zur Durchführung einer optischen Kohärenztomographie, inbesondere einer spektralen optischen Kohärenztomographie, ausgebildet ist.

4. Untersuchungsvorrichtung nach Anspruch 3, wobei die dritte Vorrichtung eine Strahlungsquelle zur Erzeugung einer Messlaserstrahlung umfasst, mit welcher der zweite Augenabschnitt abtastbar und eine Vielzahl von Abtastwerten bestimmbar ist, wobei anhand der Abtastwerte die tiefenaufgelöste Darstellung des zweiten Augenabschnitts erzeugbar ist, und die Untersuchungsvorrichtung ein Umlenkelement, insbesondere einen Spiegel, zur Einkopplung der Messlaserstrahlung der dritten Vorrichtung in einen Strahlengang der ersten Vorrichtung umfasst.

5. Untersuchungsvorrichtung nach Anspruch 4, wobei zur Bestimmung des zweiten Augenabschnitts eine räumliche Lage des Umlenkelements variierbar ist.

6. Untersuchungsvorrichtung nach Anspruch 5, wobei die dritte Vorrichtung eine weitere Strahlungsquelle zur Erzeugung einer Pilotlaserstrahlung sowie einen Koppler zur Einkopplung der Pilotlaserstrahlung in einen Strahlengang der Messlaserstrahlung umfasst, sodass der mit der Messlaserstrahlung abzutastende zweite Augenabschnitt durch eine Variation der räumlichen Lage des Umlenkelements sowie durch Beobachtung der entsprechend auf dem Auge zu beobachtenden Pilotlaserstrahlung präzise bestimmbar ist.

7. Untersuchungsvorrichtung nach Anspruch 6, wobei die dritte Vorrichtung ein den Koppler aufweisendes Interferometer sowie ein Spektrometer umfasst, die Messlaserstrahlung von dem Koppler in einen Messstrahl und einen Referenzstrahl aufteilbar ist, mit dem Koppler eine Interferenzstrahlung erzeugbar ist, indem der vom Auge reflektierte Messstrahl und der von einem weiteren Umlenkelement reflektierte Referenzstrahl in dem Koppler zur Überlagerung bringbar ist, und die Interferenzstrahlung zur spektralen Analyse zum Spektrometer führbar ist.

8. Untersuchungsvorrichtung nach einem der Ansprüche 1 bis 7, wobei die zweite Vorrichtung eine Lichtquelle, einen ersten und einen zweiten Strahlengang, einen Bildsensor, eine Auswerteeinheit sowie im ersten Strahlengang eine erste Optik und im zweiten Strahlengang eine zweite Optik umfasst, wobei ein von der Lichtquelle ausgesandter Lichtstrahl via den ersten Strahlengang mit einem ersten Umlenkelement zum Auge führbar ist, der vom Auge reflektierte Lichtstrahl via ein zweites Umlenkelement zum Bildsensor führbar und eine vom Bildsensor erzeugte Bildinformation mit der Auswerteeinheit auswertbar ist.

9. Untersuchungsvorrichtung nach einem der Ansprüche 1 bis 8, wobei sie als Faseroptik aufgebaut ist.

10. Untersuchungsvorrichtung nach einem der Ansprüche 1 bis 8, wobei sie als geometrische Optik aufgebaut ist.

11. Untersuchungsvorrichtung nach Anspruch 3, bei welcher für die Abbildung ein Binokular der ersten Untersuchungsvorrichtung und für eine Beleuchtung die Beleuchtungseinheit der zweiten Untersuchungsvorrichtung verwendbar ist, sodass dem Benutzer bei einer Betrachtung des Auges durch einen Binokulartubus des Binokulars eine stereoskopische und reflexarme Beobachtung des Auges, insbesondere eines Fundus des Auges, zur Verfügung stellbar ist.

12. Untersuchungsvorrichtung, insbesondere nach einem der Ansprüche 1 bis 11, wobei die Untersuchungsvorrichtung eine Auswerteeinheit und mindestens drei Lichtquellen, vorzugsweise LEDs, umfasst und die Lichtquellen in einer Ebene ringförmig um eine Frontlinse der ersten Vorrichtung und konzentrisch zu einem Messstrahl der dritten Vorrichtung angeordnet sind, wobei die Ebene insbesondere senkrecht zu einer optischen Achse der Frontlinse angeordnet ist.

13. Untersuchungsvorrichtung nach Anspruch 12, wobei zur Messung eines Abstands zwischen einer Sehachse und einem Pupillenschwerpunkt des Auges mit der Auswerteeinheit ein Pupillenschwerpunkt, aus den an einem Tränenfilm des Auges reflektierten Lichtstrahlen der Lichtquellen ein einer Position der Sehachse entsprechendes Zentrum und aus dem Pupillenschwerpunkt und dem Zentrum der Abstand berechnet werden kann.

14. Untersuchungsvorrichtung, insbesondere nach einem der Ansprüche 1 bis 13, wobei die Untersuchungsvorrichtung eine Anzeigeeinheit umfasst, auf welcher Patientendaten, der zu messende Augenbereich, softwaremässig erzeugte Marken als Positionierhilfen und/oder Messergebnisse darstellbar sind.

15. Untersuchungsvorrichtung nach Anspruch 14, wobei auf der Anzeigeeinheit als Positionierhilfe ein Kreis mit einem variablen Druchmesser darstellbar ist, wobei der Durchmesser bei steigender Zuverlässlichkeit der Messsignale verkleinerbar ist,

16. Untersuchungsvorrichtung nach Anspruch 12, wobei in der Ebene ringförmig um die Frontlinse **12** der ersten Vorrichtung und konzentrisch zum Messstrahl der dritten Vorrichtung mindestens 5 Lichtquellen angeordnet sind und die Auswerteeinheit zur Messung einer Krümmung einer Hornhautvorderfläche des Auges ausgebildet ist.

17. Verfahren zur Untersuchung eines Auges mit einer Untersuchungsvorrichtung, wobei mit einer ersten Vorrichtung eine Abbildung des Auges erzeugt wird, indem das Auge mit einer Beleuchtungseinheit beleuchtet und mit einem Mikroskop die Abbildung erzeugt wird, **dadurch gekennzeichnet, dass** mit einer zweiten Vorrichtung eine Abbildung eines ersten Augenabschnittes erzeugt wird, indem der erste Augenabschnitt mit einem Lichtstrahl sequentiell abgetastet wird, und dass mit einer dritten Vorrichtung eine tiefenaufgelöste Abbildung eines zweiten Augenabschnittes erzeugt wird, indem mit der dritten Vorrichtung eine Schnittansicht des zweiten Augenabschnittes parallel zu einer optischen Achse der Untersuchungsvorrichtung erzeugt wird.
